# EUROPEAN PATENT APPLICATION

(11) **EP 1 681 029 A1**
(43) Date of publication of application: **19.07.2006**
(21) Application number: 05000937.2
(22) Date of filing: 18.01.2005
(51) Int. Cl.: A61B 19/00

(54) **Apparatus and process for manipulating medical instruments**

(71) Applicant: Technische Universität München, 80333 München (DE)
(72) Inventor: Jung, Christoph, 85386 Eching (DE); Lauer, Wolfgang, 96049 Bamberg (DE)
(74) Representative: Graf von Stosch, Andreas

(57) **Abstract**

The object to provide a system and a method for manipulating medical instruments that allows that substantially all movements of the instrument or tool will be executed with the same preciseness, is being solved by the apparatus (1) according to the invention for manipulating medical instruments comprising a guiding component (2) for guiding an instrument carrying unit (7); a rotating means (3) for rotating that guiding component; a supporting means (4a,4b) for holding and fixing that rotating means whereby that guiding component is aligned along a circular shaped body.

## Description

The present invention relates to an apparatus for manipulating medical instruments and a process for manipulating a medical instruments.

It is known in the prior art to use medical robotic systems. For example the US-Patent 6,102,850 describes a system that includes a pair of a surgical instruments that are coupled to a pair of robotic arms. The instruments have end effectors that can be manipulated to hold and suture tissue. The robotic arms are coupled to a pair of master handles by a controller. The handles can be moved by the surgeon to produce a corresponding movement of the end effectors. The movement of the handles is scaled so that the end effectors have a corresponding movement that is different, typically smaller, than the movement performed by the hands of the surgeon. The scale factor is adjustable so that the surgeon can control the resolution of the end effector movement. The movement of the end effector can be controlled by input button, so that the end effector only moves when the button is depressed by the surgeon. The input button allows the surgeon to adjust the position of the handles without moving the end effector, so that the handles can be moved to a more comfortable position. The system may also have a robotically controlled endoscope which allows the surgeon to remotely view the surgical site. A cardiac procedure can be performed by making small incisions in the patient's skin and inserting the instruments and endoscope into the patient. The surgeon manipulates the handles and moves the end effectors to perform a cardiac procedure such as a coronary artery bypass graft.

Prior art document US 5,807,378 is directed to manipulator assembly for holding and manipulating a surgical instrument in a telerobotic system. The assembly comprises a base fixable by passive or power driven positioning devices to a surface, such as an operating table, and an instrument holder movably mounted on the base. The instrument holder comprises a chassis and an instrument support movably mounted on the body and having an interface engageable with the surgical instrument to releasably mount the instrument to the instrument holder. A drive assembly is operatively coupled to the instrument holder for providing the instrument with at least two degrees of freedom. The instrument holder is separable from the base and the drive assembly to that the holder can be sterilized. The assembly further includes a force sensing element mounted distal to the holder and the drive assembly for detecting forces exerted on the surgical instrument and providing feedback to the surgeon.

US 2003/0216715 A1 describes a robotic surgical systems, devices, and methods that include selectably associatable master/slave pairs, often having more manipulator arms than will be moved simultaneously by the two hands of a surgeon. Four manipulator arms can support an image capture device, a left hand tissue manipulation tool, a right hand tissue manipulation tool, and a fourth surgical instrument, particularly for stabilizing, retracting, tool change, or other functions benefiting from intermittent movement. The four or more arms may sequentially be controlled by left and right master input control devices. The fourth arm may be used to support another image capture device, and control of some or all of the arms may be transferred back-and-forth between the operator and an assistant. Two or more robotic systems each having master controls and slave manipulators may be coupled to enable cooperative surgery between two or more operators.

The known prior art systems comprise a support structure, an instrument and a controlling unit. The cinematic of the instrument which is considered the active cinematic and the cinematic of the support structure which is considered the passive cinematic enable the system seven degrees of freedom at the tip of the instrument or tool.

Three degrees of freedom might be realised within the human or animal body. The remaining four degrees of freedom will be realised outside the human or animal body and are executed by means of the supporting structure. The three degrees of freedom can be divided in two rotatable degrees of freedom and one longitudinal movement degree of freedom of the instrument or tool.

All of the drives of the known system are located out of the human or animal body in order to enable the system to be of a minimal size at the front end, i.e. the tip of the instrument or tool. The known system realises its movements by means of wires that transform the moving force converted by guide pulleys and other mechanical gears.

The known prior art system has several disadvantages. Beside the voluminous dimensions of the support structure and the controlling device, the main disadvantage is the lack of precision of movements, if the system is completely unfolded, i.e. the arms holding the instruments at their distal ends are completely stretched.

The arms of the known system comprise several members that are connected via rotatable joints to enable rotational and longitudinal movements. In order to reach from one side of an operating table where typically the known system is located to the other half of the operating table, the arms of the robotic system have to be manipulated into a protruding position having the medical instrument or tool at their distal end and the support structure along with the gears and electrical drives at their proximate end. While the instrument or tool is being located in those protruding positions the known systems show the disadvantage of imprecise movements due to the amplified effect of micro movements in the respective arm members that are closer to the support structure at the proximate end.

The impreciseness of minimal movements, however, can be detrimental if high precision surgery is demanded such as neuro surgery on nerve cords or mirco surgery on blood vessels. Although the impreciseness of the movements derive from the mechanical components, it is not possible to eliminate them completely since such impreciseness may only be compensated to a certain extend by means of using stronger material, heavier components or other self damping means. Since on the other hand lightweight construction to reduce the inertia and instant activation response is an essential object of surgical instruments, a compromise between preciseness and lightweight construction is very hard to achieve.

Furthermore, the impreciseness of movements of the instrument or tool depend on the position of the instrument or tool. The closer the instrument or tool is to the proximate end of the arm the higher is the preciseness of the movements. Additionally, horizontal movements of the instrument or tool shown lower preciseness than vertical movements, due to the nature of the type of activation of the vertical and horizontal movements. While the horizontal movements are controlled by wires and gears that are activated by devices that are located in the distanced support unit, vertical movements of the instrument or the tool are initiated at the distal end of the arms and have therefore less variation causing impreciseness.

In light of the mentioned disadvantages of the prior art, it is an object of the present invention to provide a system and a method for manipulating medical instruments that allows that substantially all movements of the instrument or tool will be executed with the same preciseness.

This object will be solved by an apparatus and method according to the invention having the features of claim 1 and 13.

Advantageous embodiments of the present invention are characterized in the dependent claims 2 to 12 and 14.

The apparatus according to the invention for manipulating medical instruments comprises a guiding component for guiding an instrument carrying unit; a rotating means for rotating that guiding component; a supporting means for holding and fixing that rotating means whereby that guiding component is aligned along a circular shaped body.

Advantageously said guiding component covers an angle of at least 90° preferably 120° along said circular shaped body.

Advantageously said guiding component comprises a curved rail to which that instrument carrying unit is attached.

Advantageously the surface of said curved rail is substantially planar. Besides a planar surface it is also possible in another embodiment of the present invention to use a surface having a cograil that engages with a cogwheel for transporting and guiding said instrument carrying unit over the guiding component.

Advantageously said instrument carrying unit is enabled to be moved onto the curved rail along with the instrument carrying unit comprising sensor means for determining the relative position of that guiding component.

In order to enable a functioning robotic system several apparatus for manipulating medical instruments according to the invention should be utilized. Hence it is another object of the present invention to provide a system for manipulating medical instruments comprising at least two of the afore-mentioned apparatus.

In order to fulfil complex movements, in particular from the fringe of the operating table to the centre, the aforementioned apparatus are preferably mounted on a longitudinal transporting means that enables at least a vertical movement of the apparatus.

In addition to a vertical longitudinal transporting means it is also advantageous to provide a second longitudinal transporting means that enables a horizontal movement of the vertical horizontal means.

Although the two longitudinal transporting means are spaced apart from the instrument carrying unit it is generally not necessary to activate them for micro inter-surgery movements of the instrument. Such micro inter surgery movements will only be carried out by the instrument, the instrument holder and the attached instrument carrying unit along with the movement of the instrument carrying unit on the guiding component.

Due to this inventive improvement a very high precision of any inter surgery movements can be maintained.

Advantageously, a longitudinal movements of the two longitudinal transporting means are so designed that a transmission slip can be avoided. The longitudinal transporting means can be designed by using high precise positioning units that are commonly known and are able to perform their preciseness repeatedly.

Advantageously, the longitudinal transporting means comprise sensor means for determining the relative position status of the transported components such as the longitudinal transporting means for horizontal movements or the rotating means which is located on the longitudinal transporting means for horizontal movements.

The present invention furthermore comprises a central controlling unit having the function of controlling all movements of the interacting gears, drives and actuator means as well as to determine the exact location of the working area of the instrument or tool.

In order to realise the requirement of controlling all movements and determining the position of the interacting components, the sensors are transmitting their signals to a processing unit. Based on the position data the central controlling unit is able to guide the instrument or tool to a predetermined position according to a stored routine, a manually or automatically desired position.

According to one aspect of the invention the processing of the positioning data is being performed by means of vectors and vector analysis. The central control unit comprises a processing unit that determines and anticipates the movements of all interacting components preferably based on a vector analysis related to the present position and the desired future position of all interacting components.

Since the inventive system utilizes for micro inter surgical movements preferably the circular shaped guiding component, the rotating means and the instrument carrying unit, the use of the supporting means for longitudinal movements in horizontal and vertical directions can normally be avoided for micro inter surgical movements.

In the event of using more than one apparatus according the invention simultaneously, the central control unit is enabled to avoid any collision between the interacting apparatus at one operating table.

The collision avoiding routine is based on a vector analyses comparison between the interacting apparatus according to the invention before and during the initiated movements.

Another aspect of the present invention is to provide a process for manipulating medical instruments having the steps of attaching an instrument on an instrument carrying unit; guiding that instrument carrying unit along an circular shaped guiding component by means of at least one actuator; rotating that guiding component by means of rotating means; transporting that rotating means vertically by means of vertical longitudinal transporting means; transporting that vertical longitudinal transporting means by means of a horizontal longitudinal transporting means.

Advantageously, the process comprises furthermore the step of controlling all movements of the components by means of sensor means to determine the relative position of the components.

When performing the process according to the present invention, the instrument carrying unit will be transported along the curved rail on the guiding component. During such movements the rod like instrument holder is positioned within the guiding component as a radius arm in an wheel with spokes. Due to that the rod like instrument holder comprises a spot along its longitudinal elongation that keeps its position throughout all movements of the rotating means and along the guiding component. This spot is called invariant point. This invariant point is the center of the curved rail mounted on the guiding component.

The tip of the instrument extends according to the individual desire of the surgical process beyond the invariant point into the human or animal body. The invariant point is considered invariant not only in relation to the curved rail and the guiding component but also invariant in relation to the rotational access of the rotating means. Due to that the invariant point remains invariant during all inter surgical movements since all inter surgical movements will be performed by the rotating means guiding component or the instrument carrying unit.

Preferably the invariant point is the access point into the human or animal body. Below the invariant point, the tool or instrument extends into the human or animal body and can be guided and moved within the space defined by a cone having its tip in the invariant point and extends below it, determined by the angle of the guiding component and rotating means as well as its length defined by the instrument carrying unit. Since the invariant point will keep its position once the instrument is placed into the human or animal body, the size of the access point and the medical trauma can be minimized and limited to the mere diameter of the guiding tube of the instrument carrying unit.

In case several apparatus according to the invention are being used, it is recommended to place the access points that constitute the invariant points of the respective apparatus on the human or animal body in a kind to maximize the operating range.

The present invention will be described in more detail by means of a preferred embodiment and a system comprising several preferred embodiments. The drawings show:
- Figure 1: a perspective view of a preferred embodiment according to the present invention;
- Figure 2: a side elevation of the preferred embodiment mounted on two longitudinal transporting means;
- Figure 3: a perspective view of the embodiment according to Figure 2;
- Figure 4: a comparative view of the preferred embodiment and its invariant point;
- Figure 5: a perspective view of three preferred embodiments constituting a system according to the invention;
- Figure 6: a close-up of the types of the various instruments.

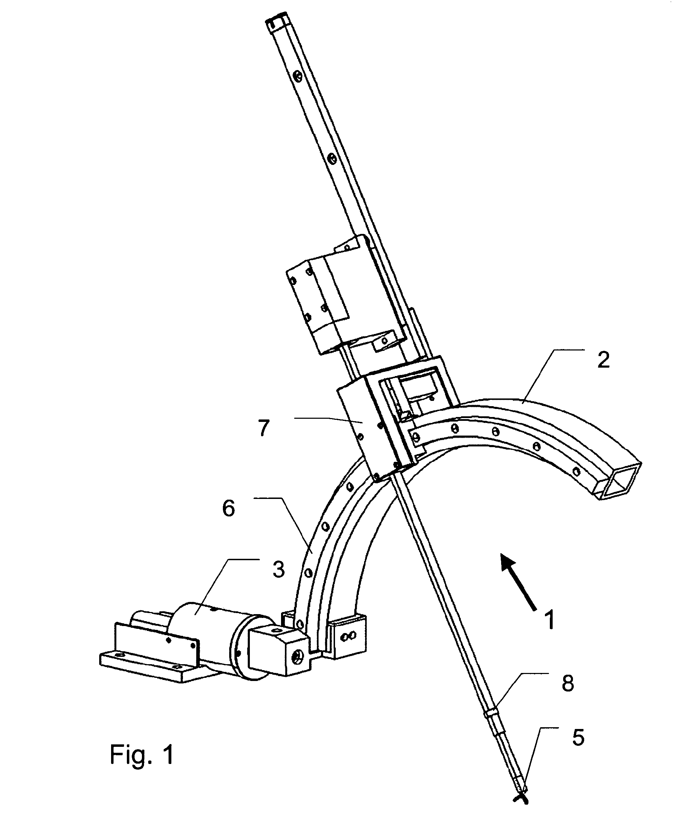

In the perspective view according to Figure 1, the apparatus for manipulating medical instruments 1 is shown comprising a guiding component 2 on which a instrument carrying unit 7 is mounted. The instrument carrying unit 7 rests on a curved rail 6, having a planar surface that enables the instrument carrying unit 7 to travel along the circular shaped body of the guiding component 2.

As shown in Figure 1 the guiding component 2 covers an angle of approximately 120°. This angle enables the instrument carrying unit 7 to reach all necessary positions with its tip 5 that may comprise a plurality of tools, instruments, optical devices or other similar units.

The guiding component 2 is attached to the rotating means 3 in order to enable a rotational movement of the entire guiding component 2. Preferably, the rotation around the axis of the rotating means 3 should cover 180°. Although the highest movement precision is required in an angle range of ± 60° off the perpendicular, it is also preferred to have high rotational precision in an angle range between ± 60° to ± 90° off the perpendicular.

The instrument carrying unit 7 extends via a tube shaped guiding element from the main component of the instrument carrying unit 7 towards the centre of the circular shaped body of the guiding component 2. The tube shaped guiding element comprises an area that is described as "invariant point" 8. This invariant point 8 is remaining its position throughout all movements of the guiding component 2, instrument carrying unit 7 and rotating means 3 to enable a minimal invasive surgery through a minimal incision in the body of the patient. The invariant point 8 is preferably located in the close vicinity to the access point into the human or animal body. In the present figures invariant point 8 (8a, 8a, 8c) is depicted as bulge located around the tube shaped guiding element. The bulge is for illustrative purposes only. In order to guarantee a smooth movement along with the tube shaped guiding element into and out of the human or animal body a unruffled area is preferred and recommended to indicate the location of the invariant point. The visibility of the invariant point is very important for the medical operation team since it may mark the depth of the penetration of the instrument into the human or animal body.

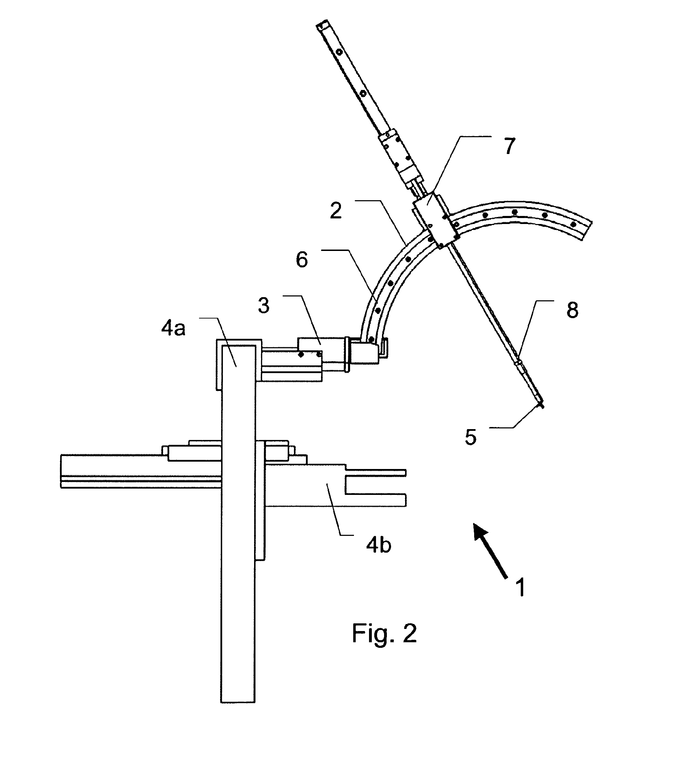

Figure 2 shows the side elevation of the preferred embodiment according to Figure 1 being attached to a longitudinal transporting means 4a to enable a vertical transportation of the entire apparatus for manipulating medical instruments 1. The longitudinal transporting means 4a is attached to a horizontal longitudinal transporting means 4b enabling the vertical longitudinal transporting means 4a to be transported horizontally.

By means by those longitudinal transporting means 4a, 4b the entire apparatus for manipulating medical instruments 1 can be adjusted and calibrated into the desired position at the operating table. Preferably the longitudinal transporting means 4a, 4 b will remain its position unchanged throughout the executed surgery.

It is, however, possible to activate the longitudinal transporting means 4a, 4b also during the surgery for performing specific movements. Due to that the longitudinal transporting means 4a, 4b are enabled to perform high precision movements.

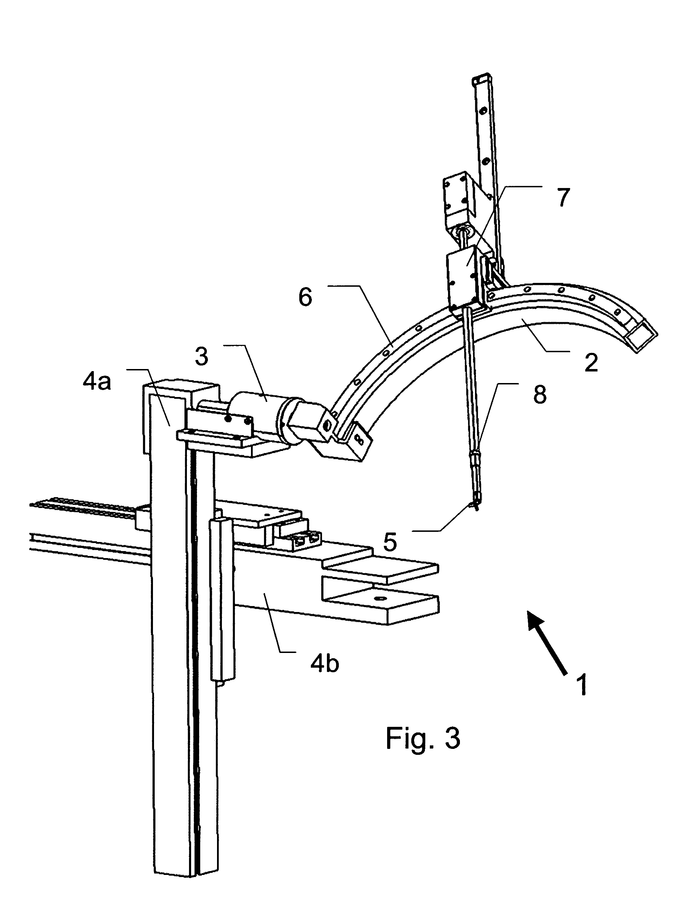

Figure 3 is a perspective view of the apparatus according to Figure 2.

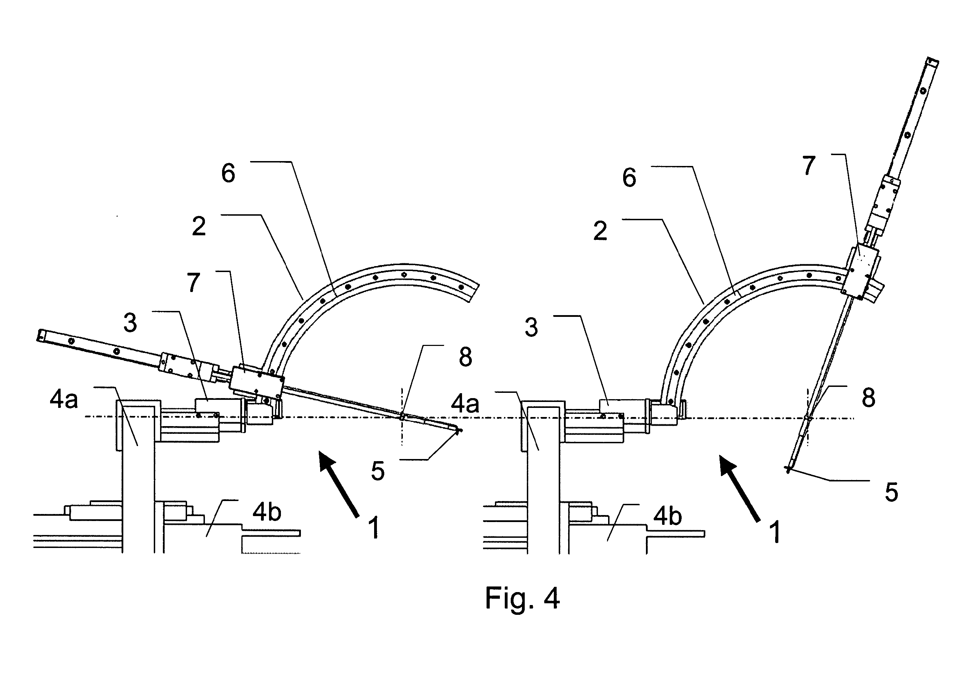

Figure 4 shows two different positions of the instrument carrying unit 7 and the tip of the instrument 5. As can be taken from Figure 4 the invariant point 8 remains at its position throughout the movement and guaranties the invariant point. This invariant point is preferably in the close vicinity of the access point to the human or animal body allowing a minimal traumatisation of the human or animal body.

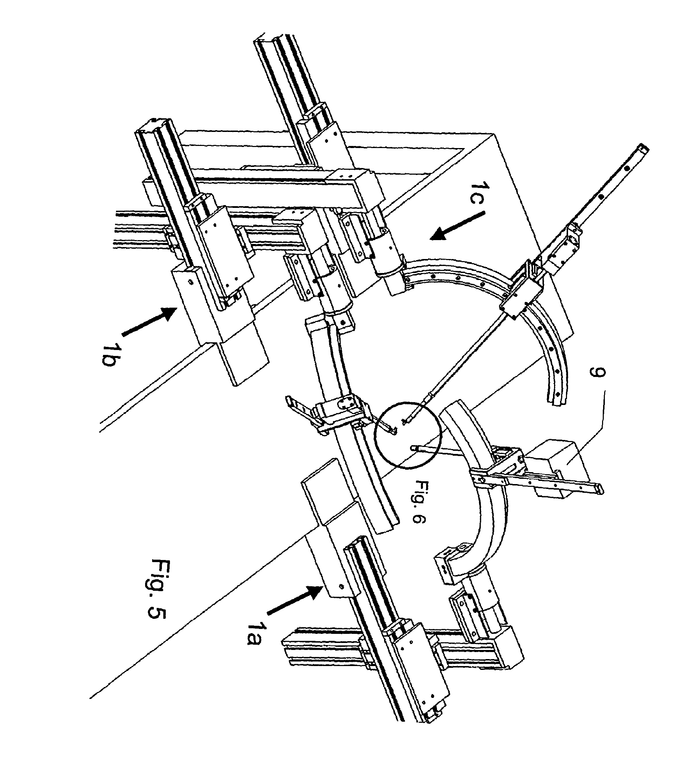

The combination of three apparatus according to the invention at one operating table is shown in Figure 5. Whereas one apparatus according to the invention 1a is located on the right hand side of the operating table, two additional apparatus according to the present invention 1c and 1b are located on the left hand side of the operating table. In order to execute complex minimally invasive surgery, it is advantageous to utilise optical detecting, amplifying or assisting devices such as endoscopes, video cameras, lights, laser devices or other assisting devices.

According to Figure 5, the apparatus 1a carries an instrument 9, for instance an optical detecting device whereas the apparatus 1b and 1c are equipped with medical instruments such as knives, pliers, clamps or other useful medical instruments.

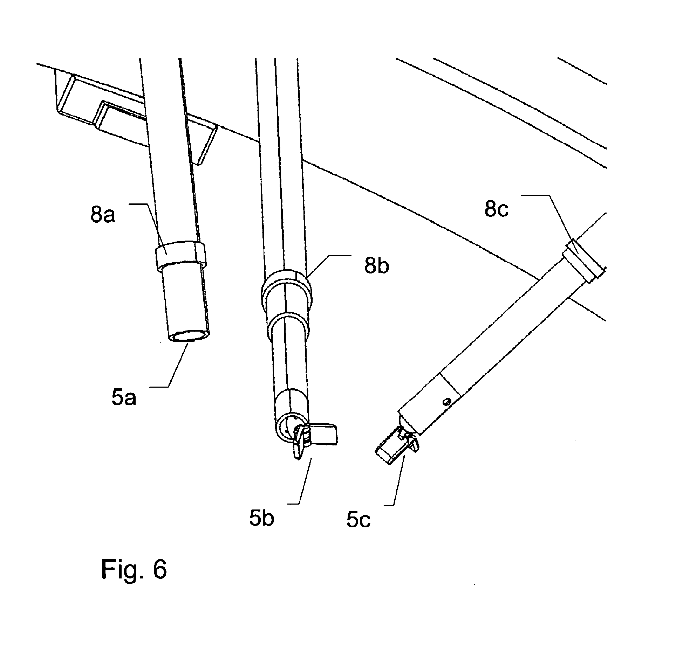

Figure 6 shows a close-up of Figure 5 enlarging the tips 5a, 5b, 5c of the respective systems 1a, 1b, 1 c of Figure 5. As can be taken from Figure 6 all invariant points 8a, 8b, 8c are in the proximity of each other and are being placed according to pre-surgery evaluations according to the diagnosis, the surgery being performed, the amount of systems being involved, as well as the location of the human or animal body at which the surgery will be performed.

## Claims

1. Apparatus for manipulating medical instruments (1), comprising:
- a guiding component (2) for guiding an instrument carrying unit (7);
- a rotating means (3) for rotating said guiding component (2);
- a supporting means (4a) for holding and fixing said rotating means (3)
**characterized in that** said guiding component (2) is aligned along a circular shaped body.

2. Apparatus (1) according to claim 1, **characterized in that** said guiding component (2) covers an angle of at least 90° along said circular shaped body.

3. Apparatus (1) according to claim 1 or 2, **characterized in that** said guiding component (2) covers an angle of 120° along said circular shaped body.

4. Apparatus (1) according to claim 2 or 3, **characterized in that** said guiding component (2) comprises a curved rail (6) to which said instrument carrying unit (7) is attached.

5. Apparatus (1) according to claim 4, **characterized in that** the surface of said curved rail (6) is substantially planar.

6. Apparatus (1) according to claim 5, **characterized in that** said curved rail (6) is laterally limited by undercuts to engage said instrument carrying unit (7).

7. Apparatus (1) according to claim 4, 5 or 6, **characterized in that** said instrument carrying unit (7) is enabled to be moved onto said curved rail (6).

8. Apparatus (1) according to any of the preceding claims, **characterized in that** the instrument carrying unit (7) comprises sensor means for determining the relative position of said guiding component (2).

9. System for manipulating medical instruments, comprising at least two of said apparatus (1 a, 1 b, 1 c) according to one of the preceding claims.

10. System for manipulating medical instruments according to claim 9, whereas each of said at least two apparatus (1a, 1b, 1c) comprises at least one longitudinal transporting means (4a, 4b).

11. System for manipulating medical instruments according to claim 10, whereas said longitudinal transporting means (4a, 4b) comprise sensor means for determining the relative position of the transported components.

12. System for manipulating medical instruments according to one of the claims 9 to 11, whereas all apparatus (1 a, 1 b, 1 c) are being control led by means of a central controlling unit.

13. Process for manipulating medical instruments having the steps of:
- attaching an instrument on an instrument carrying unit (7);
- guiding said instrument carrying unit (7) along a circular shaped guiding component (2) by means of at least one actuator;
- rotating said guiding component (2) by means of rotating means (3);
- transporting said rotating means (3) vertically by means of vertical longitudinal transporting means (4a, 4b);
- transporting said vertical longitudinal transporting means (4a, 4b) by means of a horizontal longitudinal transporting means (4a, 4b).

14. Process for a manipulating medical instruments according to claim 13, furthermore comprising the step of:
- controlling all movements of said components by means of sensor means to determine the relative position of said components.
